**Europäisches Patentamt**

**European Patent Office**

**Office européen des brevets**

(19)

(11) Veröffentlichungsnummer: **0 026 479**
**B1**

# (12) EUROPÄISCHE PATENTSCHRIFT

(45) Veröffentlichungstag der Patentschrift:
**13.02.85**

(21) Anmeldenummer: **80105829.8**

(22) Anmeldetag: **25.09.80**

(51) Int. Cl.⁴: **A 61 N 1/36**, A 61 B 5/05

(54) **Elektromedizinisches Gerät.**

(30) Priorität: **27.09.79 DE 2939234**

(43) Veröffentlichungstag der Anmeldung:
**08.04.81 Patentblatt 81/14**

(45) Bekanntmachung des Hinweises auf die Patenterteilung:
**13.02.85 Patentblatt 85/7**

(84) Benannte Vertragsstaaten:
**AT BE CH FR GB LI NL SE**

(56) Entgegenhaltungen:
**EP - A - 0 001 806**
**AT - B - 306 916**
**DD - A - 2 514**
**DE - B - 1 228 354**
**DE - B - 2 331 680**
**DE - B - 2 810 046**
**US - A - 3 791 373**
**US - A - 4 068 669**

(73) Patentinhaber: **Siemens Aktiengesellschaft, Berlin und München Wittelsbacherplatz 2, D-8000 München 2 (DE)**

(72) Erfinder: **Welgert, Kurt, Grillparzerstrasse 26, D-8510 Fürth (DE)**
Erfinder: **Hudek, Karl, verstorben (DE)**
Erfinder: **Hudek, Amanda, Galsbühlstrasse 22, D-8520 Erlangen (DE)**
Erfinder: **Hudek, Kurt, Galsbühlstrasse 22, D-8520 Erlangen (DE)**
Erfinder: **Hudek, Gerd, Galsbühlstrasse 22, D-8520 Erlangen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein elektromedizinisches Gerät für die Reizstrombehandlung, das zur Bestimmung des den Patientenkörper durchfließenden Ausgangsstromes einen Meßwiderstand aufweist, an dem der Spannungsabfall gemessen und zur Anzeige des Ausgangsstromes einer Anzeigeeinheit zugeführt wird, und das ferner zur Steuerung des den Patientenkörper als Belastungswiderstand durchfließenden Ausgangsstromes einen Stromformgenerator aufweist, der über ein Einstellglied an die Basis eines Transistors angeschlossen ist, dessen Kollektor-Emitterstrecke von dem den Patientenkörper durchfließenden Ausgangsstrom durchflossen wird und der über einen definierten Widerstand an Massepotential angeschlossen ist, dergestalt, daß der den Patientenkörper durchfließenden Ausgangsstrom bei sich änderndem Belastungswiderstand stromkonstant ausgeregelt wird.

Die Verwendung von Stromkonstantstufen bei der Reizstrombehandlung hat den Vorteil, daß sich während der Behandlung ändernde Patientenwiderstände, die eine Änderung des Stromes bewirken können, automatisch ausgeregelt werden. Gleichzeitig muß der tatsächlich fließende Patientenstrom kontinuierlich überwacht und angezeigt werden. Insbesondere bei Verwendung von impulsförmigen Reizströmen (beispielsweise bei dreieckförmigen Strömen) sollen auch jeweils die Spitzenwerte der Reizstromimpulse erfaßbar sein.

Üblicherweise wird zur möglichst genauen Strommessung im Patientenstromkreis ein Widerstand eingeschaltet, an dem der Spannungsabfall gemessen wird, der wiederum als stromproportionales Meßsignal auf ein Anzeigegerät gegeben wird. Zur Anzeige werden beispielsweise Gasentladungsröhren verwendet. Nachteilig war dabei bisher, daß die Meßwertverarbeitungs- und Anzeigeeinheiten einen eigenen, unter Umständen hohen Betriebsspannungsbedarf hatten.

In der europäischen Patentanmeldung 0 001 806 ist bereits ein elektromedizinisches Reizstromgerät der eingangs genannten Art beschrieben, bei dem in Abhängigkeit des Belastungswiderstandes, also des Patientenwiderstandes, die Betriebsspannung von einem Ausgangswert mit steigendem Belastungswiderstand zu höheren Werten geregelt wird. Die Regelzeitkonstante ist dabei so abgestimmt, daß bei einer Widerstandsänderung, die eine vorgegebene Schwelle überschreitet, die Änderung der Betriebsspannung weniger rasch erfolgt als die Änderung des Belastungswiderstandes. Dadurch wird verhindert, daß bei einer schnellen Veränderung des Belastungswiderstandes, wie sie z. B. bei einem Abgleiten der Elektroden vom Patienten vorkommen kann, hohe Stromdichten auf der Haut am Applikationsort entstehen. Solche Unfälle könnten zu Verbrennungen führen. Bemerkenswert ist, daß die bekannten Anzeigeeinrichtungen auf dem hohen Potential des Betriebsspannungszweiges liegen.

Geht man davon aus, daß bei Reizstromgeräten der eingangs genannten Art mit vergleichsweise hohen Betriebsspannungen von einigen hundert Volt gearbeitet wird, so ergibt sich aufgrund des eigenen Betriebsspannungsbedarfs für die mit dem Betriebsspannungszweig verbundenen Meß- und Anzeigegeräte eine entsprechende Potentialhöhe. Ähnliches gilt sinngemäß auch für die in der Meßwertverarbeitungsschaltung verwendeten aktiven Bauelemente, wie Operationsverstärker u. dgl. Zwar ist die absolute Betriebsspannung hierfür vergleichsweise klein, jedoch sind die Anforderungen an die Spannungsstabilität hoch. Dies macht bei einem hohen Grundpotential, das aufgrund der Stromkonstantregelung in gewissen Grenzen veränderbar ist, einen zusätzlichen schaltungstechnischen Aufwand zur Spannungsstabilisierung notwendig.

Aufgabe der Erfindung ist es, ein elektromedizinisches Gerät der eingangs genannten Art so auszugestalten, daß der Spannungsabfall zur Bestimmung des Patientenstroms (Ausgangsstrom) an Widerständen gemessen wird, die auf — im Vergleich zur Betriebsspannung — niedrigem Grundpotential oder niedrigem Potential gegen Masse liegen.

Diese Aufgabe wird nach einer ersten Ausführungsform erfindungsgemäß dadurch gelöst, daß in bekannter Weise der Meßwiderstand zwischen Betriebsspannungspotential und dem Patientenkörper liegt, daß die beiden vor und nach dem Meßwiderstand liegenden Spannungsabnahmepunkte über identische, aus Teilwiderständen zusammengesetzte Spannungsteiler an Massepotential gelegt sind, und daß die an den beiden identischen Spannungsteilern abgegriffene Spannung über einen Differenzverstärker auf die Anzeigeeinheit gegeben ist, wobei die an Massepotential liegenden Teilwiderstände der Spannungsteiler wesentlich kleiner sind als ihre mit dem Meßwiderstand verbundenen Teilwiderstände.

Nach einer zweiten Ausführungsform wird diese Aufgabe dadurch gelöst, daß der an Massepotential angeschlossene Widerstand als Meßwiderstand vorgesehen ist, daß zur Bestimmung des den Patientenkörper durchfließenden Ausgangsstroms der Spannungsabfall am Meßwiderstand und der Spannungsabfall an einem definierten Vorwiderstand in der Basisleitung des Transistors erfaßt und einem Rechenglied zugeführt werden, in dem die Differenz der beiden erfaßten Spannungsabfälle gebildet wird, und daß das Rechenglied mit Widerständen solcher Widerstandswerte beschaltet ist, daß seine Ausgangsspannung proportional zum den Patientenkörper durchfließenden Ausgangsstrom ist.

Die Erfindung kann also im wesentlichen in zwei Alternativen ausgebildet sein: Wenn man, wie beim Stand der Technik, von einem definierten Meßwiderstand im Betriebsspannungszweig ausgeht, werden nach der ersten Ausführungsform die an den Spannungsabnahmepunkten liegenden hohen Absolut-Potentiale über die identischen Spannungsteiler bis nahe an Massepotential simultan heruntergeteilt. Dabei kann die sich zwischen den beiden identischen Widerstandszweigen ergebende Spannung, die dem Spannungsabfall am Meßwiderstand entspricht, über einen Differenzverstärker auf das Anzeigegerät gegeben werden.

In der zweiten vorzugsweise angewendeten Ausführungsform wird der Spannungsabfall an Widerständen in den nicht Betriebsspannung führenden Zweigen erfaßt und über das Rechenglied zur Berechnung des patientenstromproportionalen Wertes auf das Anzeigegerät gegeben. Wird hierbei zur Stromkonstantregelung ein Transistor in Emitterschaltung verwendet, läßt sich der betreffende Spannungsabfall unmittelbar an Widerständen in der nicht an Betriebsspannung liegenden Emitterstrecke bzw. Basisstrecke des Transistors messen.

Durch die potentialmäßige Entkopplung ergeben sich insgesamt erhebliche meßtechnische Vereinfachungen. Zusätzliche Wicklungen am Spannungstransformator mit separaten geregelten Netzteilen sind nicht mehr erforderlich. Die Meßwertverarbeitungseinheiten werden von der stabilisierten Versorgungsspannung des Stromformgenerators gespeist. Insbesondere Anzeigegeräte mit hoher Versorgungsspannung können unmittelbar mit der Betriebsspannung als Spannungsversorgung beaufschlagt werden.

Weitere Einzelheiten und Vorteile der Erfindung ergeben sich aus der nachfolgenden Figurenbeschreibung von Ausführungsbeispielen anhand der Zeichnungen in Verbindung mit den Unteransprüchen. Es zeigt

Fig. 1 ein Reizstromgerät mit einer Stromkonstantstufe und Patientenstrommeßkreis nach dem Stand der Technik sowie

Fig. 2 und 3 zwei verschiedene Ausführungsbeispiele der Erfindung mit vereinfachter Patientenstrommessung.

In den Figuren sind identische Teile mit den gleichen Bezugszeichen versehen.

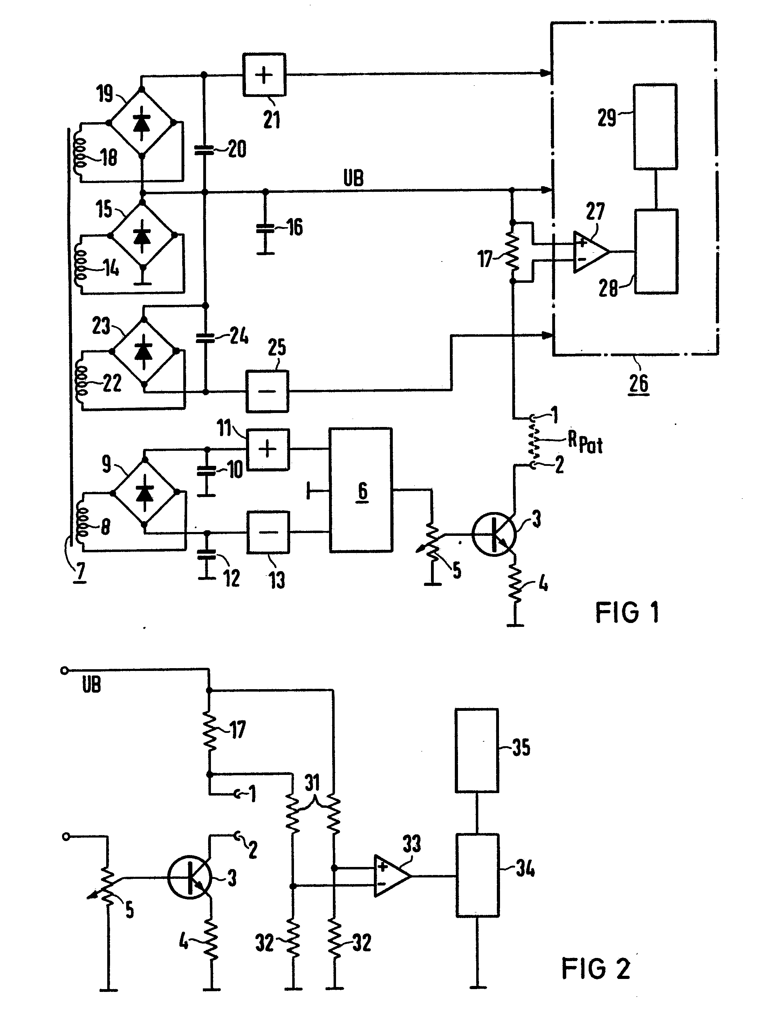

In Fig. 1 bedeuten 1 und 2 zwei Ausgangsklemmen des Reizstromgerätes, zwischen denen über Elektroden ein Patient anschließbar ist, dessen Widerstand durch die Bezeichnung $R_{Pat}$ angedeutet ist. Die Patientenausgangsklemme 1 ist an Betriebsspannung $U_B$, beispielsweise etwa 200 V, angeschlossen, während die Ausgangsklemme 2 über einen Transistor 3 in Emitterschaltung und einen Emitterwiderstand 4 mit Widerstandswert $R_E$ auf Massepotential liegt. Die Basis des Transistors 3 wird über eine Steuerleitung von einem Intensitätseinstellglied 5 angesteuert, das an einem Generator 6 angeschlossen ist. Der Generator 6 ist über eine Wicklung 8 an die Sekundärseite eines Transformators 7 angekoppelt. Von der Sekundärwicklung 8 wird eine Versorgungsspannung von ±15 V gegenüber Massepotential über eine Gleichrichterbrücke 9 mit nachgeschalteten Glättungskondensatoren 10 und 12 und Regeleinheiten 11 und 13 auf den Stromformgenerator 6 gegeben.

Die Betriebsspannung $U_B$ wird ebenfalls von der Sekundärseite des Ausgangstransformators 7 geliefert. Der Wicklung 14 ist eine Gleichrichterbrücke 15 mit Glättungskondensator 16 nachgeschaltet, die die notwendige Betriebsspannung $U_B$ liefert. Im Betriebsspannungszweig ist vor der Patientenausgangsklemme 1 in Reihe mit dem Patientenwiderstand ein definierter Meßwiderstand 17 mit Widerstandswert $R_{Meß}$ angeordnet, an dem der Spannungsabfall abgegriffen und auf einen Signalverarbeitungsschaltkreis 26 gegeben wird. Der Schaltkreis 26 besteht im wesentlichen aus einem Operationsverstärker 27 für die Meßspannung einer Aufbereitungseinheit 28 für die Meßwerte sowie einer Anzeigeeinheit 29. Mit der Aufbereitungseinheit 28 können beispielsweise Spitzen- oder Mittelwerte des Patientenstromes $I_{Pat}$ erfaßt und angezeigt werden. Als Anzeigeeinheit 29 werden speziell Gasentladungsröhren verwendet.

Operationsverstärker 27, Aufbereitungseinheit 28 und Anzeigeeinheit 29 benötigen jeweils spezifische Versorgungsspannungen. Insbesondere Gasentladungsröhren als Anzeigeeinheiten 29 haben einen Spannungsbedarf von etwa 200 V. Zur Erzeugung der jeweiligen Versorgungsspannung gegenüber dem Potential des Betriebsspannungszweiges weist daher der Transformator 7 sekundärseitig weitere Wicklungen 18 und 22 auf, über die Gleichrichterbrücken 19 und 23 mit Glättungskondensatoren 20 und 24 sowie Regeleinheiten 21 und 25 wird der entsprechende Spannungsbedarf der Meßwertbearbeitungsschaltung 26 zugeführt. Für Gasentladungsröhren als Anzeigeeinheit 29 wird ausgehend vom Betriebspotential von einigen hundert Volt gegen Massepotential zusätzlich eine Versorgungsspannung von ca. +200 V erzeugt. Die dafür notwendige weitere Sekundärwicklung mit Gleichrichter ist in Fig. 1 nicht dargestellt.

In der Fig. 2 entsprechen die Bezugszeichen 1 bis 5 und 17 denen der Fig. 1. Von den Spannungsabnahmepunkten am Meßwiderstand 17 wird nun aber die jeweils dort anliegende Spannung über zwei identische Spannungsteiler aus Widerständen 31 und 32 auf Massepotential gelegt. Mit den Spannungsteilern 31 und 32 kann also aus dem Betriebspotential ein gewünschter Wert nahe Massepotential abgeleitet werden. Entsprechend dem Teilerverhältnis des Spannungsteilers wird der Spannungsabfall am Meßwiderstand 17 geteilt. Die zwischen beiden Widerstandszweigen auftretende Differenzspannung wird mit einem Operationsverstärker 33 gemessen und zur Weiterverarbeitung auf eine Meßwertverarbeitungseinheit 34 gegeben. Nachgeschaltet ist eine entsprechende Anzeigeeinheit 35.

Wenn mit den Spannungsteilern 30 und 31 das Betriebspotential z. B. auf ein Zwanzigstel heruntergeteilt wird, so ergibt sich für die Meßspannung ebenfalls ein entsprechend verkleinerter Wert des Spannungsabfalls am Meßwiderstand $R_{Meß}$ bei niedrigem Absolut-Potential. Dieser Wert kann aber im Operationsverstärker 33, der speziell eine hohe Gleichtaktunterdrückung aufweist, störungsfrei verstärkt werden. Die Spannungsversorgung für den Operationsverstärker 39 ist die gleiche wie zur Versorgung des Stromformgenerators.

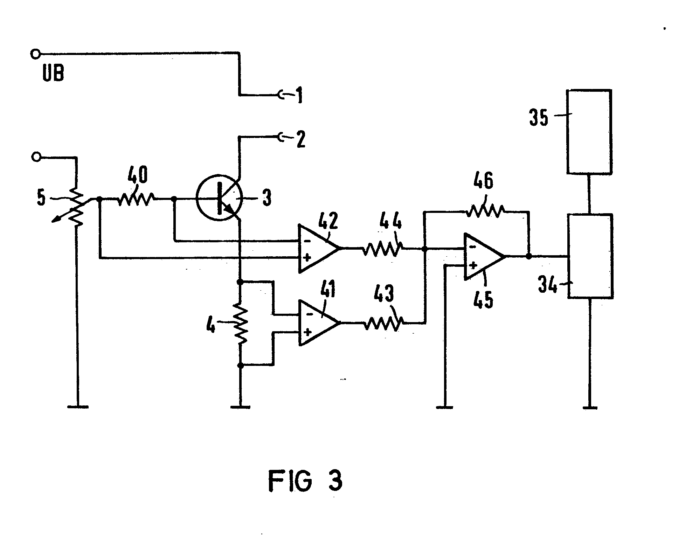

In der Fig. 3 bedeutet (neben den bereits definierten Bezugzeichen 1 bis 5) 40 einen zusätzlichen Widerstand mit Widerstandswert $R_B$ als Basisvorwiderstand in der Steuerleitung des Transistors 3. Aufgrund der Knotenregel ergibt sich der Patientenstrom $J_{Pat}$ zu

$$J_{Pat} = J_E - J_B, \qquad (2)$$

wobei $J_E$ den über den Emitterwiderstand 4 sowie $J_B$ den über den Basisvorwiderstand 40 fließenden Strom kennzeichnen. Es werden jeweils der Spannungsabfall am Emitterwiderstand 4 und am Basisvorwiderstand 40, die beide auf niedrigem Potential liegen, abgegriffen und auf Operationsverstärker 41 bzw. 42 gegeben. Die Ausgangsspannungen gelangen über nachgeschaltete Widerstände 43 bzw. 44 gemeinsam auf den invertierenden Eingang eines Umkehraddierverstärkers 45, der mit einem Widerstand 46 rückgekoppelt ist. Der nicht invertierende Eingang des Verstärkers 45 ist an Massepotential angeschlossen. Das Ausgangssignal $U_A$ des Umkehraddierverstärkers 45 wird wiederum der Meßwertverarbeitungseinheit 34 und Anzeigeeinheit 35 zugeführt.

Geht man von einer solchen rechnerischen Verarbeitung der Meßspannungen aus, so ergibt sich am Ausgang des Umkehraddierverstärkers 45 ein Meßsignal von:

$$U_A = \frac{R_3}{R_2} \cdot J_E \cdot R_E - \frac{R_3}{R_1} \cdot J_B \cdot R_B, \qquad (3)$$

wobei $R_2$ den Widerstand 43, $R_1$ den Widerstand 44, $R_3$ den Widerstand 46 bedeuten. Wählt man nun

$$R_1 = \frac{R_B}{R_E} \cdot R_2, \qquad (4)$$

so ergibt sich:

$$U_A = \frac{R_3}{R_2} \cdot R_E \cdot (J_E - J_B). \qquad (5)$$

Dieser Spannungswert ist also ein direkt proportionales Maß für den Patientenstrom $J_{Pat}$ nach Formel (2).

Bei der Erfindung sind die Meßwertverarbeitungseinheit 34 sowie die verwendeten Operationsverstärker jeweils von der stabilisierten Versorgungsspannung des Stromformgenerators 6 gegenüber Massepotential gespeist. Die als Anzeigeeinheit 35 speziell verwendeten Gasentladungsröhren können unmittelbar von der Betriebsspannung $U_B$ versorgt werden.

## Patentansprüche

1. Elektromedizinisches Gerät für die Reizstrombehandlung, das zur Bestimmung des den Patientenkörper durchfließenden Ausgangsstromes einen Meßwiderstand (17) aufweist, an dem der Spannungsabfall gemessen und zur Anzeige des Ausgangsstroms einer Anzeigeeinheit (35) zugeführt wird, und das ferner zur Steuerung des den Patientenkörper als Belastungswiderstand durchfließenden Ausgangsstroms einen Stromformgenerator (6) aufweist, der über ein Einstellglied (5) an die Basis eines Transistors (3) angeschlossen ist, dessen Kollektor-Emitterstrecke von dem den Patientenkörper durchfließenden Ausgangsstrom durchflossen wird und der über einen definierten Widerstand (4) an Massepotential angeschlossen ist, dergestalt, daß der den Patientenkörper durchfließende Ausgangsstrom bei sich änderndem Belastungswiderstand stromkonstant ausgeregelt wird, dadurch gekennzeichnet, daß der Meßwiderstand (17) zwischen Betriebsspannungspotential ($U_B$) und dem Patientenkörper liegt, daß die beiden vor und nach dem Meßwiderstand (17) liegenden Spannungsabnahmepunkte über identische, aus Teilwiderständen zusammengesetzte Spannungsteiler (31, 32) an Massepotential gelegt sind, und daß die an den beiden identischen Spannungsteilern (31, 32) abgegriffene Spannung über einen Differenzverstärker (33) auf die Anzeigeeinheit (35) gegeben ist, wobei die an Massepotential liegenden Teilwiderstände (32) der Spannungsteiler (31, 32) wesentlich kleiner sind als ihre mit dem Meßwiderstand (17) verbundenen Teilwiderstände (31) (Fig. 2).

2. Elektromedizinisches Gerät nach Anspruch 1, dadurch gekennzeichnet, daß in jedem Spannungsteiler zwei ohmsche Widerstände (31, 32) in Serie geschaltet sind (Fig. 2).

3. Elektromedizinisches Gerät für die Reizstrombehandlung, das zur Bestimmung des den Patientenkörper durchfließenden Ausgangsstromes einen Meßwiderstand (4) aufweist, an dem der Spannungsabfall gemessen und zur Anzeige des Ausgangsstroms einer Anzeigeeinheit (35) zugeführt wird, und das ferner zur Steuerung des den Patientenkörper als Belastungswiderstand durchfließenden Ausgangsstroms einen Stromformgenerator (6) aufweist, der über ein Einstellglied (5) an die Basis eines Transistors (3) angeschlossen ist, dessen Kollektor-Emitterstrecke von dem den Patientenkörper durchfließenden Ausgangsstrom durchflossen wird und der über einen definierten Widerstand (4) an Massepotential angeschlossen ist, dergestalt, daß der den Patientenkörper durchfließende Ausgangsstrom bei sich änderndem Belastungswiderstand stromkonstant ausgeregelt wird, dadurch gekennzeichnet, daß der an Massepotential angeschlossene Widerstand (4) als Meßwiderstand vorgesehen ist, daß zur Bestimmung des den Patientenkörper durchfließenden Ausgangsstroms der Spannungsabfall am Meßwiderstand (4) und der Spannungsabfall an einem definierten Vorwiderstand (40) in der Basisleitung des Transistors (3) erfaßt und einem Rechenglied (43 bis 46) zugeführt werden, in dem die Differenz der beiden erfaßten Spannungsabfälle gebildet wird, und daß das Rechenglied (43 bis 46) mit Widerständen (43, 44, 46) solcher Widerstandswerte beschaltet ist, daß seine Ausgangsspannung ($U_A$) proportional zum den Patientenkörper durchfließenden Ausgangsstrom ($J_{Pat}$) ist (Fig. 3).

4. Elektromedizinisches Gerät nach Anspruch 3, dadurch gekennzeichnet, daß das Rechenglied ein Umkehraddierverstärker (45) ist, auf dessen invertierenden Eingang beide Spannungsabfälle geleitet sind (Fig. 3).

5. Elektromedizinisches Gerät nach Anspruch 4, dadurch gekennzeichnet, daß das Rechenglied (43 bis 46) ein über einen Widerstand (46) rückgekoppelter Umkehraddierverstärker (45) ist, dessen nichtinvertierender Eingang auf Massepotential liegt und dessen invertierendem Eingang der Spannungsabfall am Widerstand (4) in der Emitterleitung über einen ersten Widerstand (43) und der Spannungsabfall am Vorwiderstand (40) in der Basisleitung des Transistors (3) über einen zweiten Widerstand (44) zugeleitet werden, wobei die Widerstandswerte des ersten und des zweiten Widerstandes (43 bzw. 44) so gewählt sind, daß ihr Verhältnis gleich dem Verhältnis der Widerstandswerte des Widerstandes (4) in der Emitterleitung und des Vorwiderstandes (40) in der Basisleitung des Transistors (3) sind (Fig. 3)

6. Elektromedizinisches Gerät nach einem der Ansprüche 1 bis 5, dadurch gekennzeichnet, daß der Transistor (3) in Emitterschaltung zwischen Patientenkörper und Massepotential geschaltet ist.

**Claims**

1. An electro-medical device for handling the stimulating current, which has a measuring impedance (17) across which the voltage-drop is measured to determine the output current flowing through the patient's body and display the output current by being fed to a display unit (35), and control of the output current flowing through the patient's body as a load impedance is provided by a current waveform generator (6) connected via a setting element (5) to the base of a transistor (3) whose collector-emitter path is traversed by the output current flowing through the patient's body and connected to earth potential via a predetermined impedance (4), such that in the event of a change in the load impedance the output current flowing through the patient's body is regulated to remain constant, characterised in that the measuring inpedance (17) is located between the operating voltage potential ($U_B$) and the patient's body, that the two voltage withdrawal points, which are arranged prior to and following the measuring resistor (17), are each connected to earth potential via identical potential dividers (31, 32) composed of subimpedances, and that the voltage between the tappings of the two identical potential dividers (31, 32) is fed via a differential amplifier (33) to the display unit (35), those sub-impedances (32) of the potential dividers (31, 32) which are connected to each potential being substantially smaller than the associated subimpedances (31) connected to the measuring impedance (17) (Fig. 2).

2. An electro-medical device as claimed in Claim 1, characterised in that in each potential divider two ohmic resistors (31, 32) are connected in series (Fig. 2).

3. An electro-medical device for handling the stimulating current, which has a measuring impedance (4) across which the voltage-drop ist measured in order to determine the output current flowing through the patient's body and display the output current by being fed to a display unit (35), and control of the output current flowing through the patient's body as a load impedance is provided by a current wave-form generator (6) connected via a setting element (5) to the base of a transistor (3) whose collector-emitter path is traversed by the output current flowing through the patient's body and connected to earth potential via a predertermined impedance (4), such that in the event of a change in the load impedance the output current flowing through the patient's body is regulated to remain constant, characterised in that the measuring impedance (4) is connected to earth potential, that the output current flowing throug the patient's body is determined by detecting the voltage-drop across the measuring impedance (4) and the voltage-drop across a predetermined series impedance (40) in the base line of the transistor (3), and are fed to a calculating unit (43 to 46) in which the difference between the two detected voltage-drops is formed, and that the calculating element (43 to 46) is connected to impedances (43, 44, 46) whose impedance values are such that its output voltage ($U_A$) is proportional to the output current ($J_{Pat}$) flowing through the patient's body (Fig. 3).

4. An electro-medical device as claimed in Claim 3, characterised in that the calculating element is an inverse adder amplifier (45) whose inverting input is supplied with the two voltage-drops (Fig. 3).

5. An electro-medical device as claimed in Claim 4, characterised in that the calculating element (43 to 46) is an inverse adder amplifier (45) having feedback via an impedance (46), its noninverting input being connected to earth potential and its inverting input being supplied with the voltage-drop across the impedance (4) in the emitter path via a first impedance (43) and with the voltage-drop across the series impedance in the base path of the transistor (3) via a second impedance (44), where the impedance values of the first and second impedances (43 and 44) are selected to be such that their ratio is equal to the ratio of the impedance values of the impedance (4) in the emitter path and the series resistor (40) in the base path of the transistor (3) (Fig. 3).

6. An electro-medical device as claimed in one of Claims 1 to 5, characterised in that the transistor (3) is connected as a common emitter stage between the patient's body and earth potential.

## Revendications

1. Appareil électromédical utilisé pour le traitement par courant de stimulation et qui comporte, pour la détermination du courant de sortie traversant le corps du patient, une résistance de mesure (17), dans laquelle la chute de tension est mesurée et est envoyée à une unité d'affichage (35) en vue de réaliser l'affichage du courant de sortie, et qui comporte en outre, pour la commande du courant de sortie traversant le corps du patient formant résistance de charge, un générateur de conformation du courant (6) qui est raccordé par l'intermédiaire d'un organe de réglage (5) à la base d'un transistor (3), dont la voie collecteur-émetteur est traversée par le courant de sortie traversant le corps du patient et qui est raccordé au potentiel de masse, par l'intermédiaire d'une résistance définie (4), de telle sorte que le courant de sortie traversant le corps du patient est réglé à une valeur constante lorsque la résistance de charge varie, caractérisé par le fait que la résistance de mesure (17) est située entre le potentiel de la tension de service (U_B) et le corps du patient, que les deux points de prélèvement de la tension, situés en amont et en aval de la résistance de mesure (17), sont raccordés au potentiel de masse par l'intermédiaire de diviseurs identiques de tension (31, 32), constitués par des résistances partielles, et que la tension prélevée sur les deux diviseurs identiques de tension (31, 32) est envoyée par l'intermédiaire d'un amplificateur différentiel (33) dans l'unité d'affichage (35), les résistances partielles (32), qui sont placées au potentiel de masse, des diviseurs de tension (31, 32) étant nettement inférieures aux résistances partielles (32) de ces diviseurs, qui sont reliées à la résistance de mesure (17) (figure 2).

2. Appareil électromédical suivant la revendication 1, caractérisé par le fait que deux résistances ohmiques (31, 32) sont branchées en série dans chaque diviseur de tension (figure 2).

3. Appareil électromédical utilisé pour le traitement par courant de stimulation et qui comporte, pour la détermination du courant de sortie traversant le corps du patient, une résistance de mesure, dans laquelle la chute de tension est mesurée et est envoyée à une unité d'affichage (35) en vue de réaliser l'affichage du courant de sortie, et qui comporte en outre, pour la commande du courant de sortie traversant le corps du patient formant résistance de charge, un générateur de conformation du courant (6) qui est raccordé par l'intermédiaire d'un organe de réglage (5) à la base d'un transistor (3), dont la voie collecteur-émetteur est traversée par le courant de sortie traversant le corps du patient et qui est raccordé au potentiel de masse, par l'intermédiaire d'une résistance définie (4), de telle sorte que le courant de sortie traversant le corps du patient est réglé à une valeur constante lorsque la résistance de charge varie, caractérisé par le fait que la résistance (4) raccordée au potentiel de masse est prévue en tant que résistance de mesure, que pour déterminer le courant de sortie traversant le corps du patient, on détecte la chute de tension aux bornes de la résistance de mesure (4) et la chute de tension aux bornes d'une résistance additionnelle définie (40) située dans le conducteur relié à la base du transistor (3) et qu'on les envoie à un circuit de calcul (43 à 46) dans lequel la différence des deux chutes de tension déterminées est formée, et que le circuit de calcul (43 à 46) est câblé avec des résistances (43, 44, 46) possédant des valeurs telles que sa tension de sortie (U_A) est proportionnelle au courant de sortie (J_{Pat}) traversant le corps du patient (figure 3).

4. Appareil électromédical suivant la revendication 3, caractérisé par le fait que le circuit de calcul est un amplificateur additionneur inverseur (45), à l'entrée inverseuse duquel sont envoyée les deux chutes de tensions (figure 3).

5. Appareil électromédical suivant la revendication 4, caractérisé par le fait que le circuit de calcul (43 à 46) est un amplificateur additionneur inverseur (45) couplé par réaction par l'intermédiaire d'une résistance (46) et dont l'entrée non inverseuse est placée au potentiel de masse et à l'entrée inverseuse duquel sont envoyées la chute de tension dans la résistance (4) située dans le conducteur raccordé à l'émetteur du transistor (3), par l'intermédiaire d'une première résistance (43), et la chute de tension dans la résistance additionnelle (40) située dans le conducteur raccordé à la base du transistor (3), par l'intermédiaire d'une seconde résistance (44), les valeurs des première et seconde résistances (43 et 44) étant choisies de manière que leur rapport est égal au rapport des valeurs de la résistance (4) situé dans le conducteur relié à l'émetteur du transistor (3) et de la résistance additionnelle (40) située dans le conducteur relié à la base du transistor (3) (figure 3).

6. Appareil électromédical suivant l'une des revendications 1 à 5, caractérisé par le fait que le transistor (3) est branché en émetteur commun entre le corps du patient et le potentiel de masse.

FIG 1

FIG 2

FIG 3